# EUROPEAN PATENT SPECIFICATION

(11) **EP 4 253 965 B1**
(45) Date of publication and mention of the grant of the patent: **10.09.2025**
(21) Application number: 22166263.8
(22) Date of filing: 01.04.2022
(51) Int. Cl.: G01N 35/00, A61B 5/1495, A61B 5/145, G01N 21/93, G01N 21/27

(54) **METHOD FOR SENSOR CALIBRATION AND IN-VITRO DIAGNOSTIC ANALYZER**
VERFAHREN ZUR SENSORKALIBRIERUNG UND IN VITRO DIAGNOSTISCHES ANALYSEGERÄT
PROCÉDÉ D'ÉTALONNAGE DE CAPTEUR ET ANALYSEUR DE DIAGNOSTIC IN VITRO

(43) Date of publication of application: 04.10.2023
(73) Proprietor: F. Hoffmann-La Roche AG, 4070 Basel (CH); Roche Diagnostics GmbH, 68305 Mannheim (DE)
(72) Inventor: Martin, Thomas, 6340 Baar (CH)
(74) Representative: Curcio, Mario

(56) References cited:
- US-A- 4 221 567
- US-A1- 2012 090 993
- US-A1- 2017 332 953

## Description

### FIELD OF THE INVENTION

The present invention refers to an automated method of calibrating a sensor located in a flow-through sensor path and requiring at least one oxygenated calibration fluid for calibration, as well as to a respective in-vitro diagnostic analyzer.

### BACKGROUND

In medicine, doctor's diagnosis and patient treatment often relies on the measurement of patient sample parameters carried out by in-vitro diagnostic analyzers. It is important that the analyzers perform correctly by providing precise and reliable measurements. Thus, it is a general requirement for in-vitro diagnostic analyzers to implement a set of quality control (QC) procedures. One of these procedures is calibration. In most cases calibration is performed using standard solutions, with known concentrations or parameters. In this way it is possible to correlate a measured signal to a quantitative result. Calibration should be performed more or less frequently depending on the system and other variable factors which may affect performance.

In blood gas and electrolyte testing, parameters are determined from a patient's sample, like the partial pressure of blood gases (pO₂, pCO₂), oxygen saturation (SO₂), pH value, electrolyte concentrations (e.g. Na⁺, K⁺, Mg₂⁺, Ca₂⁺, Li⁺, Cl⁻), bicarbonate values (HCO₃⁻), the concentration of metabolites (e.g. glucose, lactate, urea, creatinine), values for hemoglobin and hemoglobin derivatives (e.g. tHb, O₂Hb, HHb, COHb, MetHb, SulfHb), bilirubin values, and hematocrit.

Typically, the parameters are determined by conductivity, electrochemical and/or optical measuring principles. Sensors based on these measuring principles and configured to measure such sample parameters may be combined, e.g. arranged sequentially in one or more flow-through sensor paths. This allows for simultaneous and/or sequential determination of a plurality of parameters from one single sample in one single test run.

Some metabolite sensors, e.g. Glucose and Lactate sensors, require the presence of oxygen to perform measurements. In order to calibrate these sensors, calibration fluids containing lactate and/or glucose respectively and also oxygen at known levels are therefore required. Glucose/Lactate and oxygen cannot however be stored together as a ready to use calibration fluid as they react with each other, thereby changing their respective contents over storage time. Therefore Glucose/Lactate calibration fluids are typically stored without oxygen, thus requiring oxygen to be added just before sensor calibration in a process called tonometry. This process typically comprises drawing a predetermined amount of deoxygenated calibration solution into a fluidic line made of a material permeable to oxygen from ambient air, e.g. made of silicone, and waiting a predetermined time for oxygen diffusion through the walls of the fluidic line into the calibration fluid until a required level of oxygenation is reached before drawing this freshly oxygenated (tonometered) calibration fluid into the sensor path and calibrating the sensor.

As several calibration fluids may be required in order for example to execute multi-point calibrations, the tonometry process may unduly extend the calibration time, if calibration fluids are tonometered sequentially. Alternatively, parallel tonometry of calibration fluids would require additional fluidic lines and possibly additional parts like additional valves, pumps and the like, thus making the design of the analyzer more complex and more expensive. US 2017/332953 A1 relates generally to systems and methods for measuring bioanalytes.

### GENERAL DESCRIPTION

It is against the above background that aspects of the present disclosure provide certain unobvious advantages and advancements over the prior art. In particular, a new automated method of calibrating a sensor located in a flow-through sensor path and requiring at least one oxygenated calibration fluid for calibration is herein disclosed that enables faster tonometry of deoxigenated calibration fluids without requiring complex and costly design changes to the in-vitro diagnostic analyzer. An in-vitro diagnostic analyzer configured to execute said automated method and presenting the same advantages is herein also disclosed.

In particular, the automated method according to claim 1 comprises transporting a predefined amount of deoxygenated calibration fluid from a fluid supply into a fluidic line between a fluid-selection valve and the sensor path, the fluidic line having inner walls, transporting ambient air into the fluidic line before and after transporting the deoxygenated calibration fluid thereby forming an isolated plug of deoxygenated calibration fluid between zones of ambient air, oscillating the deoxygenated calibration fluid plug back and forth within a predefined length of the fluidic line comprised between the valve and the sensor path for a predefined number of times and at a predefined speed, thereby causing reciprocating fluid film tailing along the inner walls of the fluidic line, and thereby facilitating oxygenation of the deoxygenated calibration fluid via oxygen uptake by the tailing fluid film from the ambient air in the fluidic line and obtaining oxygenated calibration fluid, transporting the oxygenated calibration fluid into the sensor path at a position where the sensor is located and calibrating the sensor. The method comprises using a pump for transporting ambient air and/or the deoxygenated calibration fluid into the fluidic line, for oscillating the deoxygenated calibration fluid plug in the fluidic line and for transporting ambient air and/or the deoxygenated or oxygenated calibration fluid into the sensor path.

The term "in-vitro diagnostic analyzer" or "IVD analyzer" as used herein refers to an automated or semi-automated analytical apparatus configured to examine samples in vitro in order to provide information for screening, diagnosis or treatment monitoring purposes. The IVD analyzer is designed and configured according to the medical area of application, the parameters to be determined and corresponding laboratory workflows. For example, in a point-of-care testing environment, IVD analyzers can vary from handheld devices with low throughput, short turn-around time and limited number of measurable parameters to compact benchtop instruments with higher throughput and higher number of measureable parameters. Such IVD analyzers are designed to detect certain types of parameters, e.g. gases, electrolytes, metabolites, clinical chemistry analytes, immunochemistry analytes, coagulation parameters, hematology parameters, etc. Depending on the parameters of interest, a variety of different analytical methods and different detection technologies can be applied. For example, in the field of blood gas and electrolyte testing, electrochemical measuring principles and/or conductivity measuring principles and/or optical detection methods are used. An IVD analyzer typically comprises a plurality of functional units, each dedicated to a specific task and cooperating with each other in order to enable automated sample processing and analysis. Such functional units may include e.g. a sample input interface for receiving a sample, a fluidic system, an analytical measurement unit or detecting unit, a fluid supply unit, and the like. One or more functional units may be integrated into a larger unit or module in order to simplify the operation of the IVD analyzer.

A "fluidic system", for example, is a system comprising one or more fluidic lines connected or connectable at least in part to each other. It may further comprise at least one pump for transporting fluids through the fluidic line(s), one or more valves, chambers, filters, membranes, and the like. In particular, the fluidic system comprises at least one fluidic line for supplying fluids to a detecting unit. A "fluidic line" can be a hollow conduit such as a tubing, a channel, a chamber or combinations thereof, comprising one or more parts, suitable for the passage of fluids in a liquid tight manner, and which can have any shape and size, but which is typically optimized to minimize internal volumes and dead volumes. The parts may be flexible, rigid or elastic or combinations thereof. The fluidic system and the fluidic line in particular are typically designed to be gastight in order to avoid affecting sample parameters during transport. Nevertheless, according to an embodiment, the fluidic line may be made at least in part of a material permeable to oxygen from ambient air, e.g. made of silicone. Using the fluidic line or part of the fluidic line between the fluid-selection valve and the sensor path for tonometry of the calibration fluid has the advantage that this segment is generally free from other fluidic junctions or connections potentially leading to mixing with other fluids.

The term "fluid-selection valve" refers to a flow-regulating device to control, redirect, restrict or stop flow and, in particular, to a switching or rotary valve, that is a multi-port valve that enables to select a fluidic connection with one or more fluid reservoirs at a time. This is typically achieved by moving one or more valve conduits to switch communication between different elements. Elements may be fluidically connected via further conduits, like pipes, tubes, capillaries, microfluidic channels and the like. For example, the valve may be integrated into a manifold comprising a channel and a respective input port for each fluid reservoir, where upon switching, e.g. by rotation, of the valve a fluidic connection is established between one fluidic reservoir at a time and e.g. a common outlet port connected or connectable to the fluidic line leading to the detecting unit. The valve may comprise other input ports for other fluids like for ambient air and for samples.

The IVD analyzer also comprises at least one pump, e.g. a peristaltic pump, syringe pump membrane pump or any other suitable pump, for transporting samples from sample containers, other fluids from the fluid supply unit, or ambient air through the at least one fluidic line and through the detecting unit. In order to oscillate the deoxygenated calibration fluid plug in the fluidic line, the pump is also configured such as the pumping direction is invertible. The pump is typically positioned downstream of the detecting unit but it may be located at any other position and may be connected to the fluidic system via further elements such as valves and switches.

A "fluid supply unit" as used herein is a module or component of the IVD analyzer comprising one or more fluid containers, and possibly also one or more waste containers where fluids circulated through the fluidic system may be disposed of at the end of the process. The term "fluid" may refer to either a gas or liquid or mixtures thereof. A fluid may be for example a sample, a reagent, a reference fluid such as a quality control fluid or a calibration fluid, a cleaning fluid, a wetting fluid, air or other gas.

Samples are typically entered into the fluidic system via a sample input interface, different from the fluid supply unit, that is another module or component of an in-vitro diagnostic analyzer typically arranged at a position conveniently accessible by an operator and configured to transfer a sample from a sample container brought up by the operator into the in-vitro diagnostic analyzer. The sample input interface may e.g. comprise a sample input port comprising an outer input-port side configured for coupling, attaching, connecting, sitting, introducing or plugging-in a sample container, e.g. of the capillary-type or syringe-type, and an inner input-port side coupled to or for coupling to one end of a sample-input conduit, the sample input conduit being for example fluidically connected at the other end to the detecting unit or to the fluid-selection valve. According to one embodiment the sample-input conduit is the same fluidic line leading from the fluid-selection valve to the detector and configured to alternately connect with the same end to the inner input port side and to the fluid-selection valve, e.g. directly to the outlet port of the fluid selection valve, or to a further port connected to the outlet port of the fluid selection valve via a further conduit, in order to alternately draw a sample from the sample input port and a fluid other than a sample through the fluid-selection valve, whereas the other end is connected to the detecting unit/sensor path. According to an embodiment the fluidic line, if acting also as sample input-conduit, may be embodied at least in part as a rigid aspiration needle configured to be coupled to the inner input-port side of the sample input port and to the outlet port of the fluid selection-valve or extension thereof. According to an embodiment ambient air can also be aspirated via the sample input port directly into the fluidic line or via a dedicated air port of the fluid-selection valve.

A "calibration fluid" is a reference or standard solution, typically provided in the fluid supply unit, that contains known values of one or more calibration substances used for calibration and that is measured under the same conditions as a biological sample. A calibration substance can be the same as a quality control (QC) substance, e.g. an analyte identical to an analyte of interest potentially present in a biological sample and being detected by a particular sensor, the concentration of which is however known, or that generates by reaction an analyte identical to an analyte of interest, the concentration of which is known, or it can be any other equivalent substance of known concentration, which mimics a sample parameter of interest or that can be otherwise correlated to a certain parameter of interest, e.g. a dye that behaves optically similarly to an analyte of interest. Typically, one or two calibration fluids are used for a one-point or two-point calibration respectively, when the sensor responds linearly to analyte concentrations. Three or more calibration fluids may be used if the calibration curve is nonlinear. In particular, calibration fluids can be provided in different levels that correspond to different concentration ranges of the calibration substances.

In particular, at least one deoxygenated calibration fluid is provided in the fluid supply according to the present disclosure. The term "deoxygenated" refers to a level of oxygen that is either zero or sufficiently low that the content of any other calibration substance in the calibration fluid does not significantly change over the storage time as a result of reaction with the oxygen contained therein. In other words the level or partial pressure of oxygen in the calibration fluid is such that the shelf life of the calibration fluid is not affected by its oxygen level. Deoxygenation may be obtained upon manufacturing by e.g. degassing the solvent used for the calibration fluid, or replacing oxygen with another inert gas and in general by executing the manufacturing process in a deoxygenated environment so that oxygen uptake from air is prevented before the calibration fluid is sealed in a liquid and gas tight fluid container. In order to maintain the level of oxygenation at low or near zero level during storage, a chemical oxygen scavenger may be used in the deoxygenated calibration fluid, e.g. in order to interact with and neutralize any oxygen eventually permeated into the fluid container during storage, but whose reaction kinetics is slow enough that it does not interfere with the process of oxygenation in the fluidic line.

A "detecting unit" according to the present disclosure is an analytical measurement unit of the IVD analyzer comprising at least one flow-through sensor path. A "flow-through sensor path" is a fluidic conduit that may comprise one or more sensors that a sample flowing through the sensor path comes in contact with, e.g. arranged sequentially along the path, e.g. a sensor for each different parameter/analyte to be detected, and may be embodied in a replaceable cartridge-like structure comprising a plurality of sensors, possibly distributed across a plurality of sensor paths. In alternative, the IVD analyzer may comprise a plurality of detecting units, each having a sensor path comprising a sensor dedicated to one parameter/analyte, and which may also be replaceable or not. A sample may thus flow into the one or more sensor paths and different parameters/analytes may be determined by respective sensors. The detecting unit may optionally comprise also a flow-through optical measurement unit. The flow-through sensor path may be an integrated part of the fluidic system of the IVD analyzer or part of a separate component such as for example a sensor cartridge fluidically connected to the fluidic system of the IVD analyzer, in a way that the at least one fluidic line and the at least one sensor path are fluidically connected.

The term "sensor" is herein generically used to indicate a detector configured to detect sample parameters by generating a correlated signal output that can be quantified and digitized. The sensor can be e.g. a biosensor, a chemical sensor or a physical sensor and is typically a part of a functional unit of an IVD analyzer, e.g. an analytical measurement unit or detecting unit. The sensor can be selective or specific with respect to one sample parameter of interest or can be configured to detect and quantify a plurality of different sample parameters of interest. Depending on the type of sensor, a sensor can comprise a plurality of sensory elements. The term "sensory element" therefore refers to a part of a sensor (e.g. to a working electrode, a reference electrode, a counter electrode) that in combination with one or more other sensory elements forms a fully functional sensor.

According to certain aspects the detecting unit comprises any one or more of a pO₂ sensor, a pCO₂ sensor, a pH sensor, one or more ion selective electrode (ISE) sensors for determining electrolyte values such Na⁺, K⁺, Ca₂⁺ and Cl⁻, one or more metabolite sensors for determining parameters such as lactate and glucose. The sensors may be e.g. respectively based on the amperometric, potentiometric or conductometric principle.

For example, a pO₂ sensor typically functions according to the Clark measurement principle. This means that oxygen diffuses through a membrane to a gold multi-wire system with negative electric potential inside of the sensor. The oxygen is reduced here, which generates an electric current that is proportional to the oxygen contained in the sample. This current is measured amperometrically.

A pCO₂ sensor typically is a Severinghouse-type sensor. This means that CO₂ diffuses through a membrane similar to the oxygen sensor. In the sensor, the CO₂ concentration changes and causes a correlated change of the pH value of an internal buffer system, which is measured potentiometrically.

A pH sensor typically comprises a pH-sensitive membrane. Depending on the pH value of the test sample, electric potential is generated at the boundary layer between the membrane and the sample. This potential can be measured potentiometrically by a reference sensor.

Na⁺, K⁺, Ca₂₊ and Cl⁻ ISE sensors typically work according to the potentiometric measuring principle. They differ only by different membrane materials that enable sensitivity for the respective electrolytes.

The glucose sensor typically makes use of glucose oxidase enzyme, by which glucose is oxidized into gluconolactone with oxygen available in the sample. The H₂O₂ that is formed in this process is determined amperometrically by a manganese dioxide/carbon electrode.

The Lactate sensor typically makes use of lactate oxidase enzyme, by which lactate is oxidized into pyruvate with oxygen available in the sample. The H₂O₂ that is formed in this process is determined amperometrically in a manner similar to the glucose sensor.

According to an embodiment, the at least one sensor is a metabolite sensor including at least one of a glucose sensor and a lactate sensor, or any other biosensor involving a reaction with oxygen.

According to an embodiment, the predefined amount of deoxygenated calibration fluid drawn into the fluidic line between the fluid-selection valve and the sensor path in order to be tonometered is a minimum volume that is then sufficient to fully cover the sensor to be calibrated within a tolerance positioning range in the sensor path. The minimum volume thus depends on the area of the sensor path inner cross-section and the length of the sensor path being occupied by the sensory elements of the sensor to be calibrated as well as on the precision of positioning of this volume of calibration fluid after oxygenation within the sensor path in correspondence of the respective sensory elements. Such precision may in turn depend on the precision of the pump and/or on tolerances within the fluidic system, possibly comprising dead volumes and/or flexible parts subject to volume changes upon pressure changes within the fluidic system, and especially also on the fact that air in the fluidic line is compressible. It may depend also on the switching speed of the fluid-selection valve and resulting fluid displacement when switching the valve. Thus the tolerance may be in a narrower or broader range of e.g. 10%, 20%, 50% or even 100% or more of the volume sufficient to fully cover the sensor to be calibrated within the sensor path depending on the particular configuration and system design. The reason for using a minimum volume rather than an excess of volume is that the smaller the volume is the faster the oxygenation can be by this method.

Drawing ambient air into the fluidic line before and after transporting the calibration fluid thereby forming an isolated plug of calibration fluid between zones of ambient air, has the advantage of introducing oxygen contained in known levels in the ambient air directly into the fluidic line and in contact with the calibration fluid plug contained in this fluidic line rather than waiting for diffusion though the walls of the fluidic line. Oscillating the calibration fluid plug back and forth causes reciprocating fluid film tailing along the inner walls of the fluidic line, thus increasing the surface of calibration fluid exposed to the air and thereby accelerating oxygenation of the calibration fluid. The term "tailing" refers to the portion of liquid adhering to the inner walls of the fluidic line by surface/capillary forces and following the main moving plug. As the plug keeps moving back and forth, the tailing portion before the advancing head of the plug is rejoined to the plug while a new tailing portion is formed behind, thereby obtaining a continuing mixing.

In genaral, the faster the speed of oscillation is, the longer the oscillation distance is, and the smaller the liquid volume is, the faster the oxygenation process is. This is because the surface to volume ratio renewably exposed to air is so maximized and thus oxygen uptake by the liquid can be very quick. In particular, oscillating at higher speeds has the advantage that more distance can be covered per time unit and also that a thicker tailing fluid film (more volume) is left behind.

Since the speed of oscillation can be limited by the speed and acceleration properties of the pump, according to an embodiment, the predefined speed for oscillating the calibration fluid in the fluidic line is a nearly maximum speed that the pump is capable of. The speed may however not be higher than a predefined speed limit, above which breaking up of the liquid plug may occur, and which should be rather avoided. Thus if, for the particular fluidic system, the maximum speed of the pump is higher than the speed limit, then the speed of oscillation is lowered accordingly.

The length of oscillation, that is the distance within the fluidic line between the end positions that the calibration fluid plug reaches during one complete oscillation cycle, also contributes to the speed of oxygenation. Thus, according to an embodiment, the predefined length of the fluidic line within which the deoxygenated calibration fluid plug is oscillated is a nearly entire length of the fluidic line, that is a length between the fluid-selection valve and the sensor path or between a port connected to an outlet port of the fluid-selection valve via a further conduit and the sensor path, excluding the junctions. In particular, by increasing the oscillation distance, the liquid plug can travel at a high speed for a longer time while the number of oscillations can be reduced. This is advantageous because every time that the liquid plug changes direction it is subject to deceleration and reacceleration, with a certain still period during switching of pump direction and until the liquid starts moving in the opposite direction, time during which the oxygen uptake by the liquid film is also reduced.

The minimum number of oscillations at the predetermined speed and within the predefined length of the fluidic line can be empirically determined by the manufacturer for the particular fluidic system, e.g. by measuring the level of oxygenation, i.e. partial pressure of oxygen, after increasing numbers of oscillations, until the required level of oxygenation of the calibration fluid is reached that is suitable for calibrating the sensor. Since the partial pressure of oxygen in the ambient air may be slightly variable, e.g. depending also on altitude, an excess of oscillations may be predetermined in order to make sure that a sufficient level of oxygenation is obtained in all cases.

By minimizing the total volume and hence the length of the liquid plug the ratio between volume of the tailing liquid film and volume of the oscillating liquid plug is higher and thus the oxygen uptake is faster. Additionally the available oscillation distance is larger if the liquid plug is smaller.

According to an embodiment, the method comprises cleaning the fluidic line and the sensor path with deoxygenated calibration fluid as a first step. This step may have the advantage of removing traces of liquids and/or contaminants from previous runs out of the fluidic system and conditioning the inner surface of the fluidic line and sensor path with the calibration fluid.

According to an embodiment, the cleaning step comprises continuous transporting of deoxygenated calibration fluid plugs alternated by air plugs through the fluidic line and the sensor path for a predetermined time and at a predetermined speed. In this case the smaller the liquid plugs and the larger the number of liquid plugs the higher the cleaning efficiency may be, while reducing the total volume of calibration fluid being used for cleaning and wasted.

The method comprises filling the fluidic line and the sensor path with air as a second step, that is emptying the fluidic line and sensor path from the calibration fluid entered in the first step. This step may coincide with the step of transporting the first plug of ambient air into the fluidic line before transporting a plug of calibration fluid into the fluidic line to be oxygenated, thereby forming the first air zone ahead of the fluid plug.

The present disclosure is also directed to an in-vitro diagnostic analyzer according to claim 8 comprising at least one sensor located in a flow-through sensor path and requiring at least one oxygenated calibration fluid for calibration. The IVD analyzer further comprises a fluid supply comprising at least one deoxygenated calibration fluid, a fluid-selection valve for selecting at least one fluid at a time, a fluidic line comprised between the valve and the sensor path, and a pump wherein the IVD analyzer further comprises a controller configured to automatically execute any of the herein disclosed method steps.

According to an embodiment, the at least one sensor is a metabolite sensor including at least one of a glucose sensor and a lactate sensor.

The controller is further configured to control the pump and the fluid-selection valve, for transporting ambient air and/or the deoxygenated calibration fluid into the fluidic line, for oscillating the calibration fluid plug in the fluidic line and for transporting ambient air and/or the deoxygenated or oxygenated calibration fluid into the sensor path, including positioning the oxygenated calibration fluid in correspondence of the respective sensor for calibration.

The term "controller" as used herein may include any physical or virtual processing device and in particular a programmable logic controller running a computer-readable program provided with instructions to perform operations in accordance with an operation plan and in particular in accordance with the method of calibrating a sensor as herein disclosed. This may include a processor, a controller, a central processing unit (CPU), a microprocessor, a microcontroller, a reduced instruction circuit (RISC), an application-specific integrated circuit (ASIC), a logic circuit, or any other circuit or processor configured to execute one or more of the functions/methods described herein.

According to an embodiment, the fluidic line is made at least in part of a material permeable to oxygen from ambient air, e.g. made of silicone.

Other and further objects, features and advantages will appear from the following description of exemplary aspects and accompanying drawings, which serve to explain the principles more in detail.

### BRIEF DESCRIPTION OF THE DRAWINGS

FIG. 1A shows schematically an in-vitro diagnostic analyzer comprising a flow-through sensor path and a first step of an automated method of calibrating a sensor located in the flow-through sensor path.
FIG. 1B shows schematically the same in-vitro diagnostic analyzer of FIG. 1A and a second step of the same automated method.
FIG. 1C shows schematically the same in-vitro diagnostic analyzer of FIG. 1A-1B and a third step of the same automated method.
FIG. 1D shows schematically the same in-vitro diagnostic analyzer of FIG. 1A-1C and a fourth step of the same automated method.
FIG. 1E shows schematically the same in-vitro diagnostic analyzer of FIG. 1A-1D and a fifth step of the same automated method.
FIG. 2 shows schematically the same in-vitro diagnostic analyzer of FIG. 1A-1E and a step of transporting a sample for analysis after sensor calibration.
FIG. 3A shows schematically the principle of calibration fluid oxygenation by the method of FIG. 1A-1E.
FIG. 3B is a continuation of FIG. 3A showing the progress of oxygenation during the process.
FIG. 3C is a continuation of FIG. 3B showing the result at the end of the process.
FIG. 4 is a variant of the process shown in FIG. 3A-3C.

Skilled artisans appreciate that elements in the figures are illustrated for simplicity and clarity and have not necessarily been drawn to scale. For example, the dimensions of some of the elements in the figures may be exaggerated relative to other elements whereas other elements may have been left out or represented in a reduced number in order to enhance clarity and improve understanding of the aspects of the present disclosure.

### DETAILED DESCRIPTION

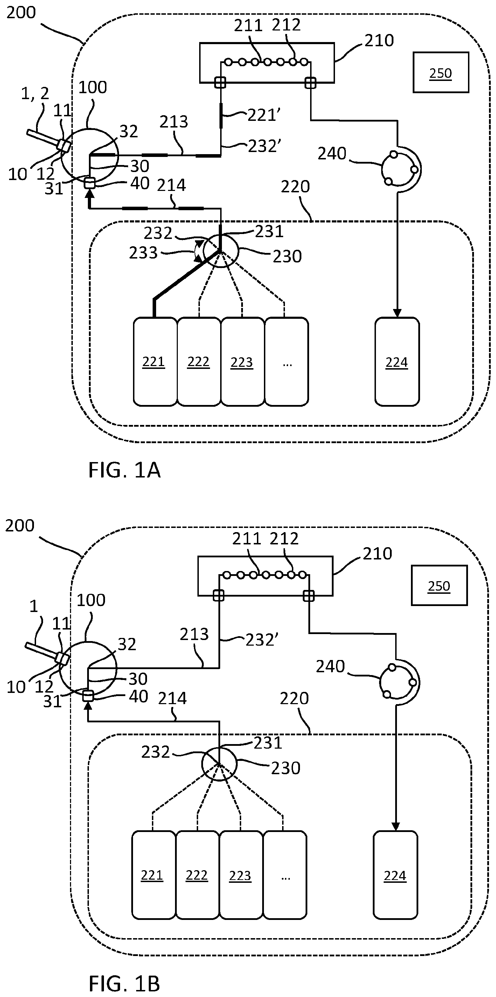

FIG. 1A to FIG. 1E taken together show schematically an example of in-vitro diagnostic analyzer 200 comprising a flow-through sensor path 211 according to the present disclosure as well as an automated method of calibrating a sensor 212 located in the flow-through sensor path 211. In particular, the in-vitro diagnostic analyzer 200 comprises a detecting unit 210 comprising a flow-through sensor path 211, the flow-through sensor path 211 comprising at least one sensor 212 requiring at least one oxygenated calibration fluid for calibration. According to an embodiment, the at least one sensor 212 is a metabolite sensor including at least one of a glucose sensor and a lactate sensor.

The IVD analyzer 200 further comprises a fluid supply 220 comprising at least one deoxygenated calibration fluid 221, among other fluids 222, 223, a fluid-selection valve 230 for selecting at least one fluid 221, 222, 223 at a time, and a fluidic line 213 comprised between the valve 230 and the sensor path 211. The IVD analyzer 200 further comprises a sample input interface 100, comprising a sample input port 10 comprising an outer input-port side 11 configured for plugging-in an open end of a sample container 1 and an inner input-port side 12. The sample container 1 is in this example a capillary-like sample container. The sample input interface 100 further comprises an aspiration needle 30 comprising an upstream end 31 and a downstream end 32. The downstream end 32 of the aspiration needle 30 is fluidically connected to the sensor path 211 via the fluidic line 213 whereas the upstream end 31 is configured to alternately couple to the inner input-port side 12 in order to aspirate a sample from a sample container 1 plugged in the outer input-port side 11 and to a fluid supply port 40 fluidically connected to an outlet port 231 of the fluid selection valve 230 via a further conduit 214. The fluidic line 213 may be however directly connected to the outlet port 231 of the fluid selection valve 230, whereas samples may be introduced via a different fluidic line separately connected to the fluid selection valve 230, for example. The fluid selection valve of FIG. 1A-1E comprises a respective input port for each fluid 221, 222, 223 comprised in a respective fluid reservoir and an air port 232, where upon switching of the valve 230 a fluidic connection is established between one fluidic reservoir 221, 222, 223 or the air port at a time and the common outlet port 231.

The IVD analyzer 200 further comprises a pump 240, such as a peristaltic pump, located downstream of the sensor path 211 and also a waste container 224, located in the fluid supply 220, where fluids circulated through the fluidic line 213 and sensor path 211 may be disposed of.

The IVD analyzer 200 further comprises a controller 250 configured to automatically execute any of the herein disclosed method steps.

In particular, FIG. 1A shows schematically a first optional step of the automated method of calibrating the at least one sensor 212 located in the flow-through sensor path 211 and requiring at least one oxygenated calibration fluid 221" for calibration. The method comprises cleaning and conditioning the fluidic line 213 and the sensor path 211 with deoxygenated calibration fluid 221 by continuously transporting deoxygenated calibration fluid plugs 221' alternated by air plugs 232' through the fluidic line 213 and the sensor path 211. This is achieved for example by continuously operating the downstream pump 240 for a predetermined time and at a predetermined speed while alternately switching connection 233 between the port fluidically connected to the calibration fluid reservoir 221 and the air port 232.

The method continues, as shown in FIG. 1B, by filling the fluidic line 213 and the sensor path 211 with air as an optional second step. This is achieved by continuously operating the downstream pump 240 for a predetermined time and at a predetermined speed while transporting air from the air port 232 connected to the outlet port 231 into the fluidic line 213 and the sensor path 211.

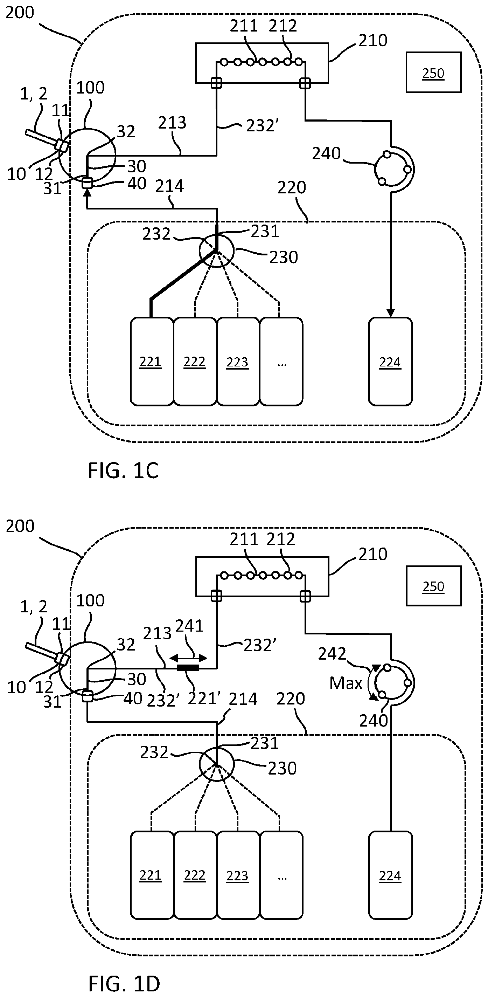

The method continues, as shown in FIG. 1C and FIG. 1D, by transporting a predefined amount of deoxygenated calibration fluid 221, from the fluid supply 220 into the fluidic line 213 between the fluid-selection valve 230 and the sensor path 211 and already contained air from the previous step, then transporting ambient air into the fluidic line 213 after transporting the deoxygenated calibration fluid 221 thereby forming an isolated plug 221' of deoxygenated calibration fluid between zones of ambient air 232', then oscillating 241 the deoxygenated calibration fluid plug 221' back and forth within a predefined length of the fluidic line 213 comprised between the valve 230 and the sensor path 211 for a predefined number of times and at a predefined speed, thereby causing reciprocating fluid film tailing along the inner walls of the fluidic line 213, thereby facilitating oxygenation of the deoxygenated calibration fluid 221 via oxygen uptake by the fluid film from the ambient air in the fluidic line 213 and thereby providing at the end of the oscillation steps a plug of oxygenated calibration fluid 221".

Oscillation 241 of the deoxygenated calibration fluid plug 221' can be achieved by repeatedly switching 242 the direction of pumping of the pump 240.

In this example, the predefined speed for oscillating the deoxygenated calibration fluid plug 221' in the fluidic line 213 is a nearly maximum speed that the pump 240 is capable of.

Also, the predefined length of the fluidic line 213 within which the calibration fluid 221' is oscillated is a nearly entire length of the fluidic line 213, including in this case also the aspiration needle 30, that is a length between the fluid supply port 40 and the sensor path 211.

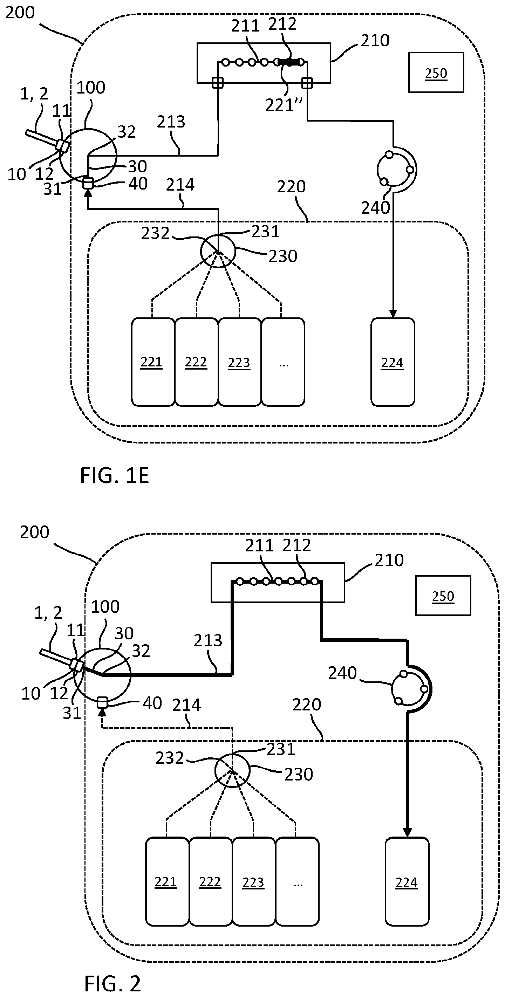

The method of calibration ends, as shown in FIG. 1E, by transporting the oxygenated calibration fluid 221" resulted from the oscillation of the deoxygenated calibration fluid plug 221' in the previous step into the sensor path 211 at a position where the sensor 212, including its sensory elements, is located and calibrating the sensor 212.

The controller 250 is configured to control the pump 240 and the fluid-selection valve 230 for transporting ambient air and/or the deoxygenated calibration fluid 221 into the fluidic line 213, for oscillating the deoxygenated calibration fluid plug 221' in the fluidic line 213 and for transporting ambient air and/or the deoxygenated or oxygenated calibration fluid 221, 221" into the sensor path, including positioning the oxygenated calibration fluid 221" in correspondence of the respective sensor 212 for calibration.

FIG. 2 shows schematically the same in-vitro diagnostic IVD analyzer 200 of FIG. 1A-1E and a step of transporting a sample 2 from the sample container 1 plugged in the outer input-port side 11 of the sample input port 10, while the upstream end 31 of the aspiration needle 30 is coupled to the inner input-port side 12 of the sample input port 10, for analysis of the sample 2 by the sensor 212 after calibration according to the method of FIG. 1A-1E. After calibration and before transporting a sample, an intermediate cleaning step with another fluid may take place (not shown). The controller 250 is in this case also configured to control the aspiration needle 30 to alternately couple to the inner input-port side 12 in order to aspirate a sample from a sample container 1 plugged in the outer input-port side 11 and to the fluid supply port 40 for performing the other steps.

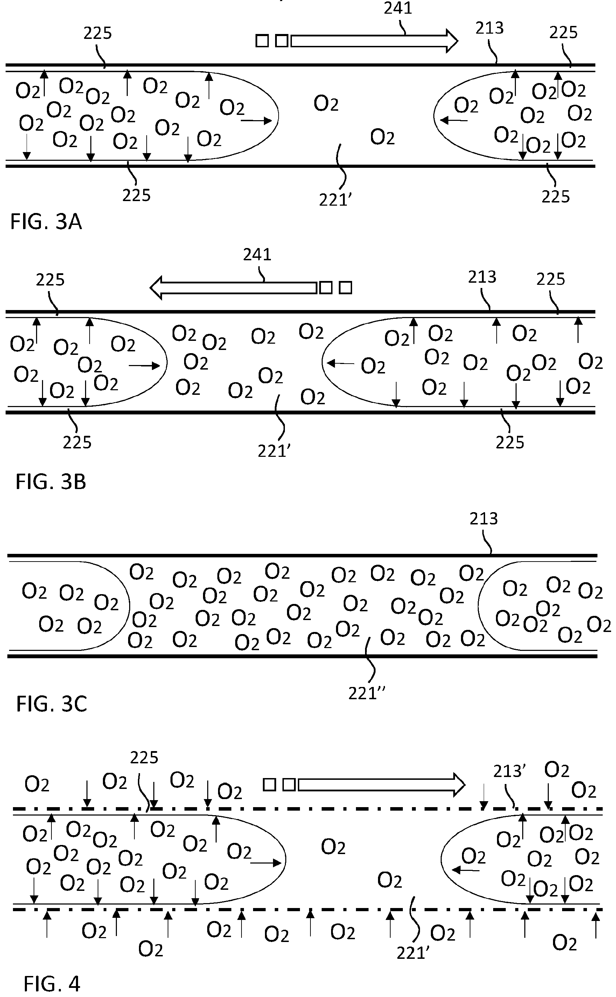

FIG. 3A-3C taken together show schematically the principle of calibration fluid oxygenation, also called tonometry, by the method of FIG. 1A-1E. In particular, the fluidic line 213 is schematically shown during the step of FIG. 4, in which a plug 221'of deoxygenated calibration fluid 221 is being oscillated 241 between zones of ambient air 232' comprising natural oxygen levels O₂ in order to be oxygenated / tonometered. Oscillating 241 the deoxygenated calibration fluid plug 221' back and forth causes reciprocating fluid film tailing 225 along the inner walls of the fluidic line 213, thus increasing the surface of liquid exposed to the air and thereby accelerating oxygenation of the deoxygenated calibration fluid plug 221'. In particular, the faster the speed of oscillation is, the longer the oscillation distance is, and the smaller the liquid volume is, the faster the oxygenation process is. This is because the surface to volume ratio renewably exposed to air is so maximized and thus oxygen uptake by the liquid can be very quick. Starting with FIG. 3A, as the deoxygenated calibration fluid plug 221' is transported from the fluid supply 220 (not shown in FIG. 3A-3C) to the fluidic line in contact with ambient air in the fluidic line 213 it starts already taking up some oxygen O₂. This can be e.g. up to 10%-50% of its final content in the oxygenated status. As the number of oscillations 241 increases, e.g. up to 30-50 or more oscillations, the oxygen uptake via the reciprocating fluid film tailing 225 increases (FIG. 3B), until a level of near saturation or equilibrium is achieved corresponding to the required level of oxygenation (partial pressure of oxygen) of the oxygenated calibration fluid 221" that is suitable for calibrating the sensor 212 and the process of oscillation is terminated (FIG. 3C), as continuing the oscillation would negligibly contribute to further increase the oxygenation level and the process would be unnecessarily prolonged. The partial pressure of oxygen in the obtained oxygenated calibration fluid 221" is typically about 80-90% of the partial pressure of oxygen in ambient air, that is about 110-170 mmHg, depending e.g. on altitude.

FIG. 4 shows a variant of the process shown in FIG. 3A-3C, with the only difference that the fluidic line 213' is made at least in part of a material permeable to oxygen O₂ from ambient air, e.g. made of silicone. Thus the combined effect of oxygen uptake via the film tailing from inside and the oxygen diffusion through the material walls from the outside may increase the speed of oxygenation even further and/or reduce the number of oscillations required to achieve the same result.

The relative abundance of oxygen "O₂" in FIG. 3A-3C and FIG. 4 is only a schematic representation, as in reality the storage capacity of oxygen (measured in mol O₂) in air is about 30 times larger than the storage capacity (saturation) in a liquid such as water under the same conditions. One liter of air in standard conditions contains 0.0093 Mol of oxygen whereas one liter of (desalinated) water at 20° C, 1 bar contains 0.000284 Mol oxygen.

Modifications and variations of the disclosed aspects are certainly possible in light of the above description. It is therefore to be understood, that within the scope of the appended claims, the invention may be practiced otherwise than as specifically devised in the above examples.

Particularly, it is to be understood that at least some of the drawings or parts are only schematic and provided as way of example only. Also the relationship between elements may be other than the one shown, whereas parts not relevant for the purpose of this disclosure have been omitted.

Also, reference throughout the preceding specification to "one aspect", "one aspect", "one example" or "an example", means that a particular feature, structure or characteristic described in connection with the aspect or example is included in at least one aspect. Thus, appearances of the phrases "in one aspect", "in one aspect", "one example" or "an example", in various places throughout this specification are not necessarily all referring to the same aspect or example.

Furthermore, the particular features, structures, or characteristics may be combined in any suitable combinations and / or sub-combinations in one or more aspects or examples.

## Claims

1. An automated method of calibrating a sensor (212) located in a flow-through sensor path (211) and requiring at least one oxygenated calibration fluid (221") for calibration, the method comprising
- transporting a predefined amount of deoxygenated calibration fluid (221) from a fluid supply (220) into a fluidic line (213) between a fluid-selection valve (230) and the sensor path (211), the fluidic line (213) having inner walls,
the automated method being **characterised by** comprising further
- transporting ambient air (232') into the fluidic line (213) before and after transporting the deoxygenated calibration fluid (221) thereby forming an isolated plug (221') of the deoxygenated calibration fluid (221) located between two zones of ambient air (232'),
- oscillating (241) the deoxygenated calibration fluid plug (221') back and forth within a predefined length of the fluidic line (213) comprised between the valve (230) and the sensor path (211) for a predefined number of times and at a predefined speed, thereby causing reciprocating fluid film tailing (225) along the inner walls of the fluidic line (213), and thereby facilitating oxygenation of the deoxygenated calibration fluid (221) via oxygen uptake by the tailing fluid film (225) from the ambient air (232') in the fluidic line (213) and obtaining oxygenated calibration fluid (221"),
- transporting the oxygenated calibration fluid (221") into the sensor path (211) at a position where the sensor (212) is located and calibrating the sensor (212),
the method comprising using a pump (240) for transporting ambient air (232') and/or the deoxygenated calibration fluid (221) into the fluidic line (213), for oscillating (241) the deoxygenated calibration fluid plug (221') in the fluidic line (213) and for transporting ambient air (232') and/or the deoxygenated or oxygenated calibration fluid (221, 221") into the sensor path (211).

2. The method according to claim 1 wherein the predefined amount of deoxygenated calibration fluid (221) is a minimum volume sufficient to fully cover the sensor (212) to be calibrated within a tolerance positioning range in the sensor path (211).

3. The method according to claim 1 or 2 wherein the predefined speed for oscillating (241) the deoxygenated calibration fluid plug (221') in the fluidic line (213) is a nearly maximum speed that the pump (240) is capable of.

4. The method according to any of the preceding claims wherein the predefined length of the fluidic line (213) within which the deoxygenated calibration fluid plug (221') is oscillated (241) is a nearly entire length of the fluidic line (213), that is a length between the fluid-selection valve (230) and the sensor path (211) or between a port (40) fluidically connected to an outlet port (231) of the fluid-selection valve (230) via a further conduit (214) and the sensor path (211).

5. The method according to any of the preceding claims comprising cleaning the fluidic line (213) and the sensor path (211) with deoxygenated calibration fluid (221) as a first step.

6. The method according to claim 5 wherein the cleaning step comprises continuous transporting of deoxygenated calibration fluid plugs (221') alternated by zones of ambient air (232') through the fluidic line (213) and the sensor path (211) for a predetermined time and at a predetermined speed.

7. The method according to claim 5 or 6 comprising filling the fluidic line (213) and the sensor path (211) with air (232') as a second step.

8. An in-vitro diagnostic IVD analyzer (200) comprising at least one sensor (212) located in a flow-through sensor path (211) and requiring at least one oxygenated calibration fluid (221") for calibration, the IVD analyzer (200) further comprising a fluid supply (220) comprising at least one deoxygenated calibration fluid (221), a fluid-selection valve (230) for selecting at least one fluid (221, 222, 223, 232') at a time, a fluidic line (213, 213') comprised between the valve (230) and the sensor path (211), and a pump (240), wherein the IVD analyzer (200) further comprises a controller (250) configured to automatically execute the method of calibrating the at least one sensor (212) according to any of the claims 1 to 7, including controlling the pump (240) and the fluid-selection valve (230) for transporting ambient air (232') and/or the deoxygenated calibration fluid (221) into the fluidic line (213, 213'), for oscillating (241) the deoxygenated calibration fluid plug (221') in the fluidic line (213) and for transporting ambient air (232') and/or the deoxygenated or oxygenated calibration fluid (221, 221") into the sensor path (211), including positioning the oxygenated calibration fluid (221") in correspondence of the respective sensor (212) for calibration.

9. The IVD analyzer (200) according to claim 8 wherein the at least one sensor (212) is a metabolite sensor including at least one of a glucose sensor and a lactate sensor.

10. The IVD analyzer (200) according to claim 8 or 9 wherein the fluidic line (213') is made at least in part of a material permeable to oxygen from ambient air.

## Patentansprüche

1. Automatisiertes Verfahren zum Kalibrieren eines Sensors (212), der sich in einem Durchfluss-Sensorpfad (211) befindet und mindestens ein sauerstoffhaltiges Kalibrierungsfluid (221") zur Kalibrierung benötigt, wobei das Verfahren Folgendes umfasst:
- Transportieren einer vordefinierten Menge an sauerstofffreiem Kalibrierungsfluid (221) von einer Fluidzufuhr (220) in eine Fluidleitung (213) zwischen einem Fluidauswahlventil (230) und dem Sensorpfad (211), wobei die Fluidleitung (213) Innenwände aufweist, wobei das automatisierte Verfahren **dadurch gekennzeichnet ist, dass** es ferner Folgendes umfasst:
- Transportieren von Umgebungsluft (232') in die Fluidleitung (213) vor und nach dem Transportieren des sauerstofffreien Kalibrierungsfluids (221), wodurch ein isolierter Pfropfen (221') des sauerstofffreien Kalibrierungsfluids (221) gebildet wird, der sich zwischen zwei Zonen von Umgebungsluft (232') befindet,
- Hin- und Herpendeln (241) des Pfropfens (221') des sauerstofffreien Kalibrierungsfluids innerhalb einer vordefinierten Länge der Fluidleitung (213), die zwischen dem Ventil (230) und dem Sensorpfad (211) enthalten ist, mit einer vordefinierten Häufigkeit und einer vordefinierten Geschwindigkeit, wodurch ein reziprozierender Fluidfilmnachlauf (225) entlang der Innenwände der Fluidleitung (213) bewirkt wird, und wodurch die Sauerstoffanreicherung des sauerstofffreien Kalibrierungsfluids (221) über die Sauerstoffaufnahme durch den Fluidfilmnachlauf (225) aus der Umgebungsluft (232') in der Fluidleitung (213) erleichtert wird und sauerstoffhaltiges Kalibrierungsfluid (221") erhalten wird,
- Transportieren des sauerstoffhaltigen Kalibrierungsfluids (221") in den Sensorpfad (211) an einer Position, an der sich der Sensor (212) befindet, und Kalibrieren des Sensors (212),
wobei das Verfahren das Verwenden einer Pumpe (240) zum Transportieren von Umgebungsluft (232') und/oder des sauerstofffreien Kalibrierungsfluids (221) in die Fluidleitung (213), zum Pendeln (241) des Pfropfens (221') des sauerstofffreien Kalibrierungsfluids in der Fluidleitung (213) und zum Transportieren von Umgebungsluft (232') und/oder des sauerstofffreien oder sauerstoffhaltigen Kalibrierungsfluids (221, 221") in den Sensorpfad (211) umfasst.

2. Verfahren gemäß Anspruch 1, wobei die vordefinierte Menge an sauerstofffreiem Kalibrierungsfluid (221) ein Mindestvolumen ist, das ausreicht, um den zu kalibrierenden Sensor (212) innerhalb eines Toleranzpositionierungsbereichs in dem Sensorpfad (211) vollständig abzudecken.

3. Verfahren gemäß Anspruch 1 oder 2, wobei die vordefinierte Geschwindigkeit zum Pendeln (241) des Pfropfens (221') des sauerstofffreien Kalibrierungsfluids in der Fluidleitung (213) eine nahezu maximale Geschwindigkeit ist, zu der die Pumpe (240) in der Lage ist.

4. Verfahren gemäß einem der vorhergehenden Ansprüche, wobei die vordefinierte Länge der Fluidleitung (213), innerhalb welcher der Pfropfen (221') des sauerstofffreien Kalibrierungsfluids pendelt (241), eine nahezu gesamte Länge der Fluidleitung (213) ist, das heißt eine Länge zwischen dem Fluidauswahlventil (230) und dem Sensorpfad (211) oder zwischen einem Anschluss (40), der über eine weitere Leitung (214) fluidisch mit einem Auslassanschluss (231) des Fluidauswahlventils (230) verbunden ist, und dem Sensorpfad (211).

5. Verfahren gemäß einem der vorhergehenden Ansprüche, umfassend das Reinigen der Fluidleitung (213) und des Sensorpfads (211) mit sauerstofffreiem Kalibrierungsfluid (221) als einen ersten Schritt.

6. Verfahren gemäß Anspruch 5, wobei der Reinigungsschritt das kontinuierliche Transportieren von Pfropfen (221') des sauerstofffreien Kalibrierungsfluids, abwechselnd mit Zonen von Umgebungsluft (232'), durch die Fluidleitung (213) und den Sensorpfad (211) für eine vorbestimmte Zeit und mit einer vorbestimmten Geschwindigkeit umfasst.

7. Verfahren gemäß Anspruch 5 oder 6, welches das Füllen der Fluidleitung (213) und des Sensorpfads (211) mit Luft (232') als einen zweiten Schritt umfasst.

8. In-vitro diagnostisches IVD-Analysegerät (200), welches mindestens einen Sensor (212) umfasst, der sich in einem Durchfluss-Sensorpfad (211) befindet und mindestens ein sauerstoffhaltiges Kalibrierungsfluid (221'') zur Kalibrierung benötigt, wobei das IVD-Analysegerät (200) ferner eine Fluidzufuhr (220), die mindestens ein sauerstofffreies Kalibrierungsfluid (221) umfasst, ein Fluidauswahlventil (230) zum Auswählen von jeweils mindestens einem Fluid (221, 222, 223, 232'), eine Fluidleitung (213, 213'), die zwischen dem Ventil (230) und dem Sensorpfad (211) enthalten ist, und eine Pumpe (240) umfasst, wobei das IVD-Analysegerät (200) ferner eine Steuerung (250) umfasst, die konfiguriert ist, um das Verfahren zum Kalibrieren des mindestens einen Sensors (212) gemäß einem der Ansprüche 1 bis 7 automatisch auszuführen, einschließlich des Steuerns der Pumpe (240) und des Fluidauswahlventils (230) zum Transportieren von Umgebungsluft (232') und/oder des sauerstofffreien Kalibrierungsfluids (221) in die Fluidleitung (213, 213'), zum Pendeln (241) des Pfropfens (221') des sauerstofffreien Kalibrierungsfluids in der Fluidleitung (213) und zum Transportieren von Umgebungsluft (232') und/oder des sauerstofffreien oder sauerstoffhaltigen Kalibrierungsfluids (221, 221") in den Sensorpfad (211), einschließlich des Positionierens des sauerstoffhaltigen Kalibrierungsfluids (221") in Übereinstimmung mit dem jeweiligen Sensor (212) zur Kalibrierung.

9. IVD-Analysegerät (200) gemäß Anspruch 8, wobei der mindestens eine Sensor (212) ein Metabolitsensor ist, der mindestens einen Glukosesensor und einen Laktatsensor einschließt.

10. IVD-Analysegerät (200) gemäß Anspruch 8 oder 9, wobei die Fluidleitung (213') mindestens teilweise aus einem Material hergestellt ist, das für Sauerstoff aus Umgebungsluft durchlässig ist.

## Revendications

1. Procédé automatisé d'étalonnage d'un capteur (212) situé dans un trajet de capteur (211) à flux traversant et nécessitant au moins un fluide d'étalonnage oxygéné (221") pour l'étalonnage, le procédé comprenant
- le transport d'une quantité prédéfinie de fluide d'étalonnage désoxygéné (221) d'une alimentation en fluide (220) dans une ligne fluidique (213) entre une vanne de sélection de fluide (230) et le trajet de capteur (211), la ligne fluidique (213) ayant des parois internes,
le procédé automatisé étant **caractérisé en ce qu'**il comprend en outre
- le transport d'air ambiant (232') dans la ligne fluidique (213) avant et après le transport du fluide d'étalonnage désoxygéné (221) formant ainsi un bouchon isolé (221') du fluide d'étalonnage désoxygéné (221) situé entre deux zones d'air ambiant (232'),
- l'oscillation (241) du bouchon de fluide d'étalonnage désoxygéné (221') d'avant en arrière au sein d'une longueur prédéfinie de la ligne fluidique (213) comprise entre la vanne (230) et le trajet de capteur (211) un nombre de fois prédéfini et à une vitesse prédéfinie, amenant ainsi le mouvement alternatif de la queue de film de fluide (225) le long des parois internes de la ligne fluidique (213), et facilitant ainsi l'oxygénation du fluide d'étalonnage désoxygéné (221) par absorption d'oxygène par le film de fluide de queue (225) de l'air ambiant (232') dans la ligne fluidique (213) et l'obtention d'un fluide d'étalonnage oxygéné (221"),
- le transport du fluide d'étalonnage oxygéné (221") dans le trajet de capteur (211) au niveau d'une position où se situe le capteur (212) et l'étalonnage du capteur (212),
le procédé comprenant l'utilisation d'une pompe (240) pour le transport d'air ambiant (232') et/ou du fluide d'étalonnage désoxygéné (221) dans la ligne fluidique (213), pour l'oscillation (241) du bouchon de fluide d'étalonnage désoxygéné (221') dans la ligne fluidique (213) et pour le transport d'air ambiant (232') et/ou du fluide d'étalonnage désoxygéné ou oxygéné (221, 221") dans le trajet de capteur (211).

2. Procédé selon la revendication 1 dans lequel la quantité prédéfinie de fluide d'étalonnage désoxygéné (221) est un volume minimal suffisant pour recouvrir entièrement le capteur (212) à étalonner au sein d'une plage de positionnement de tolérance dans le trajet de capteur (211).

3. Procédé selon la revendication 1 ou 2 dans lequel la vitesse prédéfinie pour l'oscillation (241) du bouchon de fluide d'étalonnage désoxygéné (221') dans la ligne fluidique (213) est une vitesse quasiment maximale dont la pompe (240) est capable.

4. Procédé selon l'une quelconque des revendications précédentes dans lequel la longueur prédéfinie de la ligne fluidique (213) au sein de laquelle le bouchon de fluide d'étalonnage désoxygéné (221') oscille (241) est une longueur quasiment entière de la ligne fluidique (213), c'est-à-dire une longueur entre la vanne de sélection de fluide (230) et le trajet de capteur (211) ou entre un orifice (40) raccordé de manière fluidique à un orifice de sortie (231) de la vanne de sélection de fluide (230) par une conduite (214) supplémentaire et le trajet de capteur (211).

5. Procédé selon l'une quelconque des revendications précédentes comprenant le nettoyage de la ligne fluidique (213) et du trajet de capteur (211) avec du fluide d'étalonnage désoxygéné (221) en tant que première étape.

6. Procédé selon la revendication 5 dans lequel l'étape de nettoyage comprend le transport en continu de bouchons de fluide d'étalonnage désoxygéné (221') alternant avec des zones d'air ambiant (232') à travers la ligne fluidique (213) et le trajet de capteur (211) pendant un temps prédéterminé et à une vitesse prédéterminée.

7. Procédé selon la revendication 5 ou 6 comprenant le remplissage de la ligne fluidique (213) et du trajet de capteur (211) avec de l'air (232') en tant que deuxième étape.

8. Analyseur de diagnostic in vitro IVD (200) comprenant au moins un capteur (212) situé dans un trajet de capteur (211) à flux traversant et nécessitant au moins un fluide d'étalonnage oxygéné (221") pour l'étalonnage, l'analyseur IVD (200) comprenant en outre une alimentation en fluide (220) comprenant au moins un fluide d'étalonnage désoxygéné (221), une vanne de sélection de fluide (230) pour la sélection d'au moins un fluide (221, 222, 223, 232') en même temps, une ligne fluidique (213, 213') comprise entre la vanne (230) et le trajet de capteur (211), et une pompe (240), dans lequel l'analyseur IVD (200) comprend en outre un dispositif de commande (250) configuré pour exécuter automatiquement le procédé d'étalonnage de l'au moins un capteur (212) selon l'une quelconque des revendications 1 à 7, comprenant la commande de la pompe (240) et de la vanne de sélection de fluide (230) pour le transport d'air ambiant (232') et/ou du fluide d'étalonnage désoxygéné (221) dans la ligne fluidique (213, 213'), pour l'oscillation (241) du bouchon de fluide d'étalonnage désoxygéné (221') dans la ligne fluidique (213) et pour le transport d'air ambiant (232') et/ou du fluide d'étalonnage désoxygéné ou oxygéné (221, 221") dans le trajet de capteur (211), comprenant le positionnement du fluide d'étalonnage oxygéné (221") en correspondance avec le capteur (212) respectif pour l'étalonnage.

9. Analyseur IVD (200) selon la revendication 8 dans lequel l'au moins un capteur (212) est un capteur de métabolite comprenant au moins un parmi un capteur de glucose et un capteur de lactate.

10. Analyseur IVD (200) selon la revendication 8 ou 9 dans lequel la ligne fluidique (213') est constituée au moins en partie d'un matériau perméable à l'oxygène de l'air ambiant.
